# EUROPÄISCHE PATENTSCHRIFT

(11) **EP 2 734 276 B1**
(45) Veröffentlichungstag und Bekanntmachung des Hinweises auf die Patenterteilung: **27.05.2015**
(21) Anmeldenummer: 12730806.2
(22) Anmeldetag: 27.06.2012
(51) Int. Cl.: B01D 1/06, B01D 1/26, B01D 1/28, C07C 17/02, B01D 1/00, C01D 1/42

(54) **VERFAHREN ZUM KONZENTRIEREN WÄSSRIGER LAUGE UND DAFÜR GEEIGNETE VORRICHTUNG**
METHOD FOR CONCENTRATING AQUEOUS LYE AND APPARATUS SUITABLE THEREFOR
PROCÉDÉ DE CONCENTRATION D'UNE LESSIVE ALCALINE AQUEUSE ET DISPOSITIF ADAPTÉ ASSOCIÉ

(30) Priorität: 21.07.2011 DE 102011108211
(43) Veröffentlichungstag der Anmeldung: 28.05.2014
(73) Patentinhaber: ThyssenKrupp Industrial Solutions AG, 45143 Essen (DE); Vinnolit GmbH & Co. KG, 84508 Burgkirchen (DE)
(72) Erfinder: BENJE, Michael, 65812 Bad Soden (DE); PETERSEN, Sven, 01217 Dresden (DE); KLEIBER, Michael, 65795 Hattersheim (DE)
(74) Vertreter: Wolff, Felix
(86) Internationale Anmeldenummer: PCT/EP2012/002688
(87) Internationale Veröffentlichungsnummer: WO 2013/010621

(56) Entgegenhaltungen:
- WO-A1-2007/031223

## Beschreibung

Die Erfindung betrifft ein Verfahren sowie eine Vorrichtung zum Aufkonzentrieren von wässriger Lauge in Anlagen zur Erzeugung von 1,2-Dichlorethan.

1,2-Dichlorethan (nachstehend "DCE") wird überwiegend als Zwischenprodukt der Herstellung von monomerem Vinylchlorid verwendet, welches wiederum als Zwischenprodukt für die Herstellung von Polyvinylchlorid eingesetzt wird. Bei der Umsetzung von DCE zu monomerem Vinylchlorid entsteht Chlorwasserstoff HCl. Dieser wird bevorzugt bei der Herstellung von DCE durch Oxychlorierung von Ethen mittels HCl und Sauerstoff eingesetzt. Ein alternativer Herstellungsweg von DCE führt über die Direktchlorierung von Ethen mittels Chlor. Bei der großtechnischen Herstellung von DCE werden beide Wege beschritten, so dass hinsichtlich des erzeugten und verbrauchten Chlorwasserstoffs eine ausgewogene Bilanz entsprechend den folgenden Reaktionsgleichungen erreicht wird: .

Cl₂ + C₂H₄ → C₂H₄Cl₂ + 218 kJ/Mol

C₂H₄Cl₂ → C₂H₃Cl + HCl - 71 kJ/Mol

C₂H₄ + 2 HCl + ½ O₂ → C₂H₄Cl₂ + H₂O + 238 kJ/Mol

Das für die Direktchlorierung benötigte Chlor wird häufig in einer in der Nähe der DCE-Anlage befindlichen Chloralkalielektrolyseanlage erzeugt. Dabei entsteht bekanntermaßen auch Natronlauge, typischerweise in einer Konzentration von ca. 30 Gew. %.

Diese Lauge stellt einen Wertstoff dar, der aber aus Gründen der Ökonomie aufkonzentriert wird. Moderne Anlagen zur Chloralkalielektrolyse sind mit einer Konzentrationseinheit für die anfallende Lauge ausgerüstet. Diese Einheit wird üblicherweise mit Wasserdampf betrieben und erzeugt typischerweise Laugen mit einer Konzentration von 50 Gew. % oder größer an Natriumhydroxid. Die erforderliche Menge an Wasserdampf zum Betrieb der Konzentrationseinheit stellt einen nicht unerheblichen Teil der Betriebskosten der Chloralkalielektrolyseanlage dar.

Es ist bereits vorgeschlagen worden, beim Eindampfen bzw. Aufkonzentrieren von Natronlauge aus der Chloralkalielektrolyse Wärme aus dem DCE-Prozess zu verwenden. DE 10 2005 044 177 A1 beschreibt ein Verfahren und eine Vorrichtung zur Nutzung der bei der Reinigung von DCE aus einer Direktchlorierungsanlage anfallenden Kondensationswärme. Dabei wird die Kondensationswärme der Brüden, die bei der destillativen Reinigung von DCE anfallen, zumindest teilweise für die Eindampfung der aus einer Chloralkalielektrolyseanlage stammenden Natronlauge eingesetzt. DE 10 2005 030 511 A1 und DE 10 2005 030 512 A1 beschreiben ein Verfahren und Vorrichtungen zur Nutzung der bei der Herstellung von DCE anfallenden Reaktionswärme. Dabei wird die Reaktionswärme aus einer Direktchlorierungsanlage zumindest teilweise für die Eindampfung der aus einer Chloralkalielektrolyseanlage stammenden Natronlauge eingesetzt. Es werden Vorrichtungen eingesetzt, die aus einem Rohrbündelwärmetauscher mit zwei festen Rohrplatten und einem NaOH-Sumpfteil bestehen, die so ausgeprägt sind, dass Natronlauge rohrinnenseitig und DCE auf der Außenseite der Rohre zu führen ist und die Einrichtungen aufweisen, Natronlauge auf das Rohrinnere aufzugeben und aufzuteilen.

WO-A-2007/031223 offenbart ein Verfahren zum Betrieb einer Destillationskolonne zur Reinigung von 1,2-Dichlorethan und zur gekoppelten Natronlaugeeindampfung.

Diese vorbekannten Verfahren und Vorrichtungen gestatten bereits eine sehr effiziente Prozessführung. Im Rahmen der Prozessoptimierung werden weitere Verfahrensverbesserungen angestrebt und der Prozess soll noch ressourcenschonender betrieben werden können.

Aufgabe der vorliegenden Erfindung ist die Bereitstellung eines Verfahrens und einer dafür geeigneten Vorrichtung zur Aufkonzentration von wässriger Lauge, wobei dieses Verfahren bzw. diese Vorrichtung im Vergleich zu bekannten Verfahren bzw. Vorrichtungen eine energieeffizientere Aufarbeitung gestattet. Außerdem soll die Vorrichtung so aufgebaut sein, dass es auf einfache Art und Weise möglich ist, bestehende Kombinationen von DCE Anlagen mit Chloralkalielektrolyseanlagen nachzurüsten.

Die vorliegende Erfindung betrifft ein Verfahren zur Nutzung der Reaktionswärme aus der Bildung von 1,2-Dichlorethan zum Aufkonzentrieren von wässriger Lauge, dadurch gekennzeichnet, dass die wässrige Lauge in mehreren Stufen aufkonzentriert wird, dass zumindest ein Teil der für das Aufkonzentrieren der wässrigen Lauge benötigten Wärme aus einer Anlage zur Herstellung von 1,2-Dichlorethan stammt und dass zumindestens ein weiterer Teil der für das Aufkonzentrieren der wässrigen Lauge benötigten Wärme aus mindestens einer der höheren Stufen der Anlage zum Aufkonzentrieren der wässrigen Lauge stammt und zum teilweisen Aufheizen der ersten Stufe verwendet wird, wobei das Verfahren mindestens zweistufig betrieben wird und folgende Schritte umfasst:
i) Erzeugung eines ersten wässrigen Laugenkonzentrats aus einer verdünnten wässrigen Lauge in einer ersten Aufkonzentriereinheit,
ii) Aufkonzentrieren des ersten wässrigen Laugenkonzentrats zu einem zweiten wässrigen Laugenkonzentrat in einer zweiten Aufkonzentriereinheit,
iii) Betrieb der zweiten Aufkonzentriereinheit zumindest teilweise mit Wärme, die aus einer Anlage zur Herstellung von 1,2-Dichlorethan stammt, und
iv) Betrieb der ersten Aufkonzentriereinheit zumindest teilweise mit Wärme, die aus der zweiten Aufkonzentriereinheit stammt, und wobei zwischen erster und zweiter Aufkonzentriereinheit eine Aufwärmeinheit für das erste wässrige Laugenkonzentrat geschaltet ist, die durch Wärme beheizt wird, die aus der Anlage zur Herstellung von 1,2-Dichlorethan stammt, und/oder die durch Wärme beheizt wird, die aus der zweiten Aufkonzentriereinheit stammt, und/oder die durch Wärme beheizt wird, die aus der Anlage zum Aufkonzentrieren von wässriger Lauge stammt, die mit einer Anlage zur Chloralkalielektrolyse kombiniert ist.

Unter wässriger Lauge ist im Rahmen dieser Beschreibung jede Lauge zu verstehen, die durch Verdampfen von Wasser aufkonzentriert werden kann. Vorzugsweise handelt es sich bei der Lauge um wässrige Alkalilauge, ganz besonders bevorzugt um wässrige Natronlauge.

Diese wässrige Alkalilauge stammt typischerweise aus einer Chloralkalielektrolyseanlage, insbesondere aus einer parallel zur DCE-Anlage vorhandenen Chloralkalielektrolyseanlage. In dieser wird eine wässrige Lauge erzeugt, welche vor einem Verkauf als Wertstoff aufkonzentriert werden muss. Typischerweise beträgt die Konzentration an Alkalihydroxid in einer aus einer Chloralkalielektrolyse stammenden wässrigen Alkalilauge etwa 30 Gew. %. Als Wertstoffe hingegen sollten wässrige Alkalilaugen einen Gehalt an Alkalihydroxid von mindestens 50 Gew. % aufweisen.

Bevorzugt wird ein Verfahren, worin die eingesetzte wässrige Lauge aus einer Chloralkalielektrolyseanlage stammt.

Unter Stufe ist im Rahmen dieser Beschreibung ein Verfahrensschritt zu verstehen, bei dem die wässrige Lauge die durch Verdampfen von Wasser aufkonzentriert wird.

Diesen Stufen vor- und/oder nachgeschaltet können Erwärmungsschritte der wässrigen Lauge oder der Konzentrate sein. So kann beispielsweise die als Ausgangsprodukt eingesetzte verdünnte wässrige Lauge zunächst vorgewärmt werden und danach in einer Aufkonzentriereinheit einer ersten Stufe konzentriert werden; oder die vorkonzentrierte wässrige Lauge kann nach der ersten Stufe nach dem Verlassen der ersten Aufkonzentriereinheit einem Erwärmungsschritt unterzogen werden und danach in einer zweiten Aufkonzentriereinheit einer weiteren Stufe unterzogen werden. Auch andere Kombinationen von Aufkonzentrierschritten und Erwärmungsschritten sind möglich. Die in diesen Schritten eingesetzten Apparate können mit Abwärme aus den vorangegangenen Schritten betrieben werden. Zumindest ein Teil der Abwärme stammt erfindungsgemäß aus der Anlage zur Herstellung von 1,2-Dichlorethan.

Das erfindungsgemäße Verfahren kann zweistufig oder insbesondere dreistufig oder noch höherstufig betrieben werden. Besonders bevorzugt werden dreistufig betriebene Verfahren.

Bevorzugt sind mindestens zweistufig betriebene Verfahren umfassend folgende Schritte:
i) Erzeugung eines ersten wässrigen Laugenkonzentrats aus einer verdünnten wässrigen Lauge in einer ersten Aufkonzentriereinheit
ii) Aufkonzentrieren des ersten wässrigen Laugenkonzentrats zu einem zweiten wässrigen Laugenkonzentrat in einer zweiten Aufkonzentriereinheit,
iii) Betrieb der zweiten Aufkonzentriereinheit zumindestens teilweise mit Wärme, die aus einer Anlage zur Herstellung von 1,2-Dichlorethan stammt und
iv) Betrieb der ersten Aufkonzentriereinheit zumindestens teilweise mit Wärme, die aus der zweiten Aufkonzentriereinheit stammt.

Besonders bevorzugt sind zweistufige Verfahren, bei denen zwischen erster und zweiter Aufkonzentriereinheit eine Aufwärmeinheit für das erste wässrige Laugenkonzentrat geschaltet ist, die vorzugsweise aus ein bis drei in Serie geschalteten Wärmetauschern besteht, und die durch Wärme beheizt wird, die aus der Anlage zur Herstellung von 1,2-Dichlorethan stammt, und/oder die durch Wärme beheizt wird, die aus der zweiten Aufkonzentriereinheit stammt, und/oder die durch Wärme beheizt wird, die aus einer Anlage zum Aufkonzentrieren von wässriger Lauge stammt, die mit einer Anlage zur Chloralkalielektrolyse kombiniert ist.

Besonders bevorzugt sind dreistufige Verfahren umfassend folgende Schritte:
i) Erzeugung eines ersten wässrigen Laugenkonzentrats aus einer verdünnten wässrigen Lauge in einer ersten Aufkonzentriereinheit
ii) Aufkonzentrieren des ersten wässrigen Laugenkonzentrats zu einem zweiten wässrigen Laugenkonzentrat in einer zweiten Aufkonzentriereinheit,
iii) Aufkonzentrieren des zweiten wässrigen Laugenkonzentrats zu einem dritten wässrigen Laugenkonzentrat in einer dritten Aufkonzentriereinheit,
iv) Betrieb der zweiten und dritten Aufkonzentriereinheit zumindestens teilweise mit Wärme, die aus einer Anlage zur Herstellung von 1,2-Dichlorethan stammt und
v) Betrieb der ersten Aufkonzentriereinheit zumindestens teilweise mit Wärme, die aus der zweiten und der dritten Aufkonzentriereinheit stammt.

Zur Erzeugung des ersten wässrigen Laugenkonzentrats aus wässriger Lauge in der ersten Aufkonzentriereinheit können beliebige dafür geeignete Vorrichtungen eingesetzt werden. Bevorzugt werden Wärmetauscher oder Verdampfer dafür verwendet.

Zumindest ein Teil der für den Betrieb der ersten Aufkonzentriereinheit benötigten thermischen Energie, beispielsweise mindestens 50 %, vorzugsweise mindestens 80 % der in dieser Einheit benötigten thermischen Energie stammt aus der oder den nachgeschalteten Aufkonzentriereinheit(en). Dabei kann es sich durch den Einsatz von Wärmetauschern gewonnene thermische Energie handeln und/oder um thermische Energie, die aus wieder komprimiertem Wasserdampf, der beim Betrieb der nachgeschalteten Aufkonzentriereinrichtungen angefallen ist, gewonnen wurde.

Zur Erzeugung des zweiten wässrigen Laugenkonzentrats aus dem ersten wässrigen Laugenkonzentrat in der zweiten Aufkonzentriereinheit können ebenfalls beliebige dafür geeignete Vorrichtungen eingesetzt werden. Bevorzugt werden Fallfilmverdampfer dafür verwendet; es können aber auch Wärmetauscher oder Verdampfer anderer Bauart verwendet werden. Zumindest ein Teil der für den Betrieb der zweiten Aufkonzentriereinheit benötigten thermischen Energie, beispielsweise mindestens 50 %, vorzugsweise mindestens 80 % der in dieser Einheit benötigten thermischen Energie wird aus flüssigem und/oder gasförmigen DCE gewonnen, das aus der DCE-Anlage stammt. Dabei kann es sich durch den Einsatz von Wärmetauschern gewonnene thermische Energie handeln, die aus dem flüssigen DCE-Zirkulationsstrom oder durch Kondensation von gasförmigem DCE gewonnen wurde, die aus der DCE-Anlage stammen.

Zur Erzeugung des dritten wässrigen Laugenkonzentrats aus dem zweiten wässrigen Laugenkonzentrat in der dritten Aufkonzentriereinheit können ebenfalls beliebige dafür geeignete Vorrichtungen eingesetzt werden. Bevorzugt werden hier ebenfalls Wärmetauscher oder Verdampfer dafür verwendet. Zumindest ein Teil der für den Betrieb der dritten Aufkonzentriereinheit benötigten thermischen Energie, beispielsweise mindestens 50 %, vorzugsweise mindestens 80 % der in dieser Einheit benötigten thermischen Energie wird durch Kondensation von gasförmigem DCE gewonnen, das aus der DCE-Anlage stammt.

In einer besonders bevorzugten Ausführungsform des erfindungsgemäßen Verfahrens wird einTeil des aus der dritten Aufkonzentriereinheit stammenden kondensierten DCE zur Beheizung der zweiten Aufkonzentriereinheit abgezweigt und mit dem aus der DCE-Anlage stammenden flüssigen DCE, dem DCE-Zirkulationsstrom, kombiniert.

Die vorliegende Erfindung betrifft darüber hinaus eine Vorrichtung zum Aufkonzentrieren von wässriger Lauge, die mit einer Anlage zur Herstellung von 1,2-Dichlorethan gekoppelt ist und welche die folgenden Elemente umfasst:
A) erste Aufkonzentriereinheit zum Aufkonzentrieren von wässriger Lauge und zur Erzeugung eines ersten wässrigen Laugekonzentrats, die mit Abwärme aus einer zweiten Aufkonzentriereinheit beheizt wird, und
B) zweite Aufkonzentriereinheit zum Aufkonzentrieren des ersten wässrigen Laugekonzentrats und zur Erzeugung eines zweiten wässrigen Laugekonzentrats, welche zumindestens teilweise mit Wärme beheizt wird, die aus einer Anlage zur Herstellung von 1,2-Dichlorethan stammt, und
C) zusätzlich neben den Elementen A) und B) eine Aufwärmeinheit C) für das erste wässrige Laugenkonzentrat, die zwischen die erste und die zweite Aufkonzentriereinheit geschaltet ist.

Bestehende DCE-Anlagen, die mit einer Chloralkalielektrolyseanlage verbunden sind, können mit der erfindungsgemäßen Vorrichtung nachgerüstet werden. Das ist besonders dann angebracht, wenn die Chloralkalielektrolyseanlage bereits mit einer Einheit zum Aufkonzentrieren von wässriger Lauge ausgerüstet ist. Die Nachrüstung mit einer erfindungsgemäßen Vorrichtung kann so ausgeführt werden, dass die aus der Chloralkalielektrolyseanlage stammende verdünnte wässrige Lauge in der erfindungsgemäßen Vorrichtung zunächst aufkonzentriert wird, beispielsweise von etwa 30 Gew. auf etwa 40 Gew. % an Alkalihydroxid, und sodann in der bereits in der Chloralkalielektrolyseanlage vorhandenen Einheit zum Aufkonzentrieren von wässriger Lauge weiter aufkonzentriert wird, beispielsweise von etwa 40 Gew. auf etwa mindestens 50 Gew. % an Alkalihydroxid. Die Wärme zum Betrieb der erfindungsgemäßen Vorrichtung stammt zumindest zum Teil aus der DCE-Anlage. Daher kann der Gesamtenergieverbrauch dieses Anlagenverbunds gesenkt werden, denn die in der Chloralkalielektrolyseanlage vorhandene Einheit muss mit insgesamt weniger Heissdampf betrieben werden, als ohne den Einsatz der erfindungsgemäßen Vorrichtung.

Bei DCE-Neuanlagen empfiehlt sich bevorzugt der Einbau einer dreistufig betriebenen erfindungsgemäßen Vorrichtung. Bei Kombinationen von Chlorelektrolyseanlage mit DCE-Anlage kann auf den Einbau einer in der Chloralkalielektrolyseanlage vorhandenen Einheit zum Aufkonzentrieren von wässriger Lauge verzichtet werden; diese Aufgabe kann vollständig von der erfindungsgmäßen Vorrichtung übernommen werden. Besonders bevorzugte erfindungsgemäße Vorrichtungen umfassen zusätzlich neben den oben beschriebenen Elementen A) und B) eine Aufwärmeinheit C) für das erste wässrige Laugenkonzentrat, die zwischen die erste und die zweite Aufkonzentriereinheit geschaltet ist, die vorzugsweise aus ein bis drei in Serie geschalteten Wärmetauschern besteht, und die durch Wärme beheizt wird, die aus der Anlage zur Herstellung von 1,2-Dichlorethan stammt, und/oder die durch Wärme beheizt wird, die aus der zweiten Aufkonzentriereinheit stammt, und/oder die durch Wärme beheizt wird, die aus einer Anlage zum Aufkonzentrieren von wässriger Lauge stammt, die mit einer Anlage zur Chloralkalielektrolyse kombiniert ist

Ganz besonders bevorzugt sind Vorrichtungen mit einer Aufwärmeinheit C), die aus drei in Serie geschalteten Wärmetauschern besteht, wovon ein Wärmetauscher durch Wärme beheizt wird, die aus der Anlage zur Herstellung von 1,2-Dichlorethan stammt, ein weiterer Wärmetauscher durch Wärme beheizt wird, die aus der zweiten Aufkonzentriereinheit stammt, und ein weiterer Wärmetauscher durch Wärme beheizt wird, die aus einer Anlage zum Aufkonzentrieren von wässriger Lauge stammt, die mit einer Anlage zur Chloralkalielektrolyse kombiniert ist.

Besonders bevorzugte erfindungsgemäße Vorrichtungen umfassen zusätzlich neben den oben beschriebenen Elementen A) und B) und C) die folgenden Elemente:
D) dritte Aufkonzentriereinheit zum Aufkonzentrieren des zweiten wässrigen Laugekonzentrats, welche zumindestens teilweise mit Wärme betrieben wird, die aus der Anlage zur Herstellung von 1,2-Dichlorethan stammt, und gegebenenfalls
E) eine Wärmetauschereinheit zur Rückgewinnung von Wärme aus der dritten Aufkonzentriereinheit, welche mit der zweiten Aufkonzentriereinheit so verschaltet ist, dass die rückgewonnene Wärme zur Vorwärmung des ersten wässrigen

Laugekonzentrats vor der zweiten Aufkonzentriereinheit genutzt werden kann. Bevorzugt werden Vorrichtungen, worin die erste Aufkonzentrierereinheit mit Wasserdampf aus einer Dampf-Kompressionseinheit beheizt wird, welche aus der zweiten Aufkonzentriereinheit und gegebenenfalls aus weiteren Aufkonzentriereinheiten stammenden Wasserdampf komprimiert.

Bevorzugt werden Vorrichtungen, worin die erste Aufkonzentriereinheit und die dritte Aufkonzentriereinheit jeweils ein oder mehrere in Serie geschaltete Wärmeaustauscher oder Verdampfer sind und worin die zweite Aufkonzentriereinheit ein Fallfilmverdampfer ist.

Bevorzugt werden Vorrichtungen, worin die erste Aufkonzentiereinheit und die dritte Aufkonzentriereinheit aus einem oder mehreren Plattenwärmetauschern gebildet wird.

Die erfindungsgemäße Vorrichtung ermöglicht einen besonders energiesparenden Betrieb einer Aufkonzentriereinrichtung für wässrige Lauge bei gleichzeitig hohem Wirkungsgrad der Anreicherung.

Nachfolgend wird die Erfindung anhand von vier Figuren beispielhaft näher erläutert, ohne dass dadurch eine Beschränkung beabsichtigt ist. Es zeigen:
- Fig. 1:: Eine Prinzipskizze einer erfindungsgemäßen Anlage (hergestellt durch Nachrüstung einer konventionellen Anlage) und des erfindungsgemäßen Verfahrens.
- Fig. 2:: Eine Prinzipskizze einer erfindungsgemäßen Anlage (als Neuanlage) und des erfindungsgemäßen Verfahrens.
- Fig. 3:: Eine Detailskizze einer zweistufigen Variante des erfindungsgemäßen Verfahrens und einer Anlage zu dessen Durchführung (gemäß dem Prinzip der Figur 1).
- Fig. 4:: Eine Detailskizze einer dreistufigen Variante des erfindungsgemäßen Verfahrens und einer Anlage zu dessen Durchführung (gemäß dem Prinzip der Figur 2).

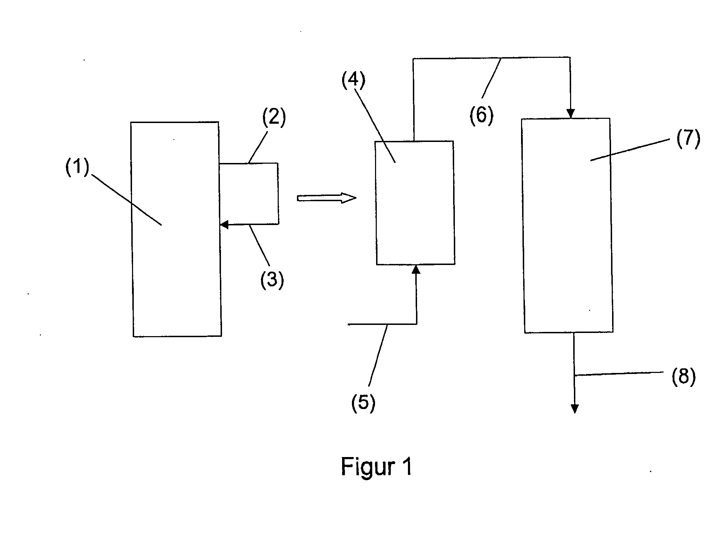

Figur 1 beschreibt das Prinzip des erfindungsgemäßen Verfahrens / der erfindungsgemäßen Vorrichtung am Beispiel der Nachrüstung einer bestehenden Anlage.

In Figur 1 ist eine DCE-Anlage (hier eine Direktchlorierungsanlage) (1) dargestellt. Aus dieser wird DCE-Dampf (2) abgezweigt und durch Kondensation wird Wärme gewonnen. Das DCE-Kondensat (3) wird in die DCE-Anlage (1) rückgeführt. Mit der gewonnenen Wärme wird eine mehrstufige Aufkonzentriervorrichtung (4) für wässrige Lauge beheizt. Im gezeigten Beispiel wird wässrige Natronlauge (5) mit einer Konzentration von etwa 30 Gew. % in die mehrstufige Aufkonzentriervorrichtung (4) eingeleitet. Die wässrige Natronlauge entstammt einer nicht dargestellten Chloralkalielektrolyseanlage welche mit einer konventionellen Aufkonzentriervorrichtung (7) für wässrige Lauge ausgestattet ist. Diese wird mit Heissdampf betrieben (nicht dargestellt). In der mehrstufigen Aufkonzentriervorrichtung (4) wird die wässrige Natronlauge (5) durch Verdampfen von Wasser aufkonzentriert und verlässt diese Vorrichtung (4) als vorkonzentrierte wässrige Natronlauge (6) mit einer Konzentration von etwa 40 Gew. %. Diese vorkonzentrierte wässrige Natronlauge (6) wird sodann in die konventionelle Aufkonzentriervorrichtung (7) eingeleitet, wo die vorkonzentrierte wässrige Natronlauge (6) durch Verdampfen von weiterem Wasser nochmals aufkonzentriert wird und die Vorrichtung (7) als wässrige Natronlauge (8) mit einer Konzentration von 50 Gew. % oder höher verlässt. Nicht dargestellt in dieser Prinzipskizze ist, dass ein Teil der für das Beheizen der mehrstufigen Aufkonzentriervorrichtung (4) benötigten Wärme aus Abwärme stammt, welche aus der konventionellen Aufkonzentriervorrichtung (7) stammt. Mit der in Figur 1 dargestellten mehrstufigen Aufkonzentriervorrichtung (4) lassen sich Verbunde aus bestehenden DCE-Anlagen und Chloralkalielektrolyseanlagen nachrüsten, welche bereits eine konventionelle Aufkonzentriervorrichtung (7) enthalten. Dazu wird die mehrstufige Aufkonzentriervorrichtung (4) wie oben beschrieben mit den bestehenden Anlagen verschaltet.

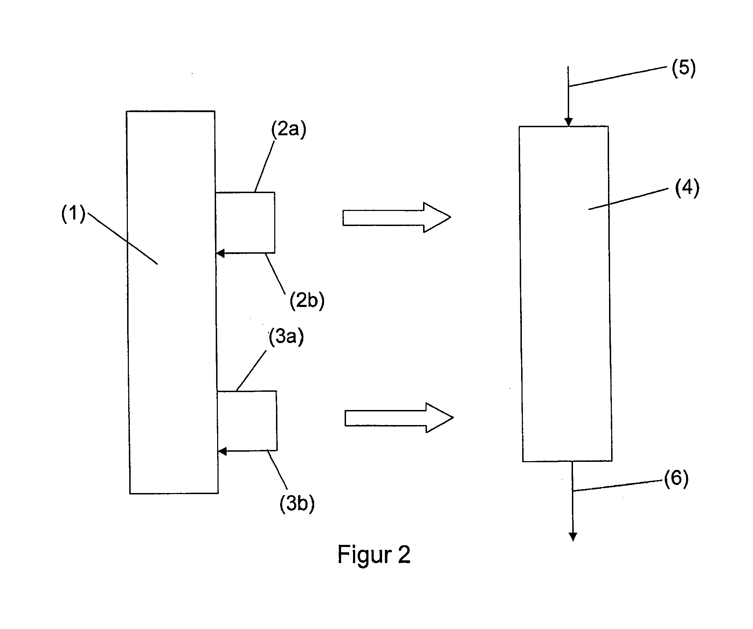

In Figur 2 ist eine DCE-Anlage (hier eine Direktchlorierungsanlage) (1) dargestellt. Aus dieser wird DCE-Dampf (2a) sowie heißes flüssiges DCE (2b) abgezweigt. Durch Kondensation des DCE-Dampfes (2a) und durch Abkühlung des flüssigen DCE (2b) wird jeweils Wärme gewonnen. Das DCE-Kondensat (3a) bzw. die abgekühlte DCE-Flüssigkeit (3b) werden in die DCE-Anlage (1) rückgeführt. Mit der gewonnenen Wärme wird eine mehrstufige Aufkonzentriervorrichtung (4) für wässrige Lauge geheizt. Im gezeigten Beispiel wird diese nicht detailliert dargestellt sondern es wird ein Blockschaltbild einer mehrstufigen Anlage gezeigt. Darin wird wässrige Natronlauge (5) mit einer Konzentration von etwa 30 Gew. % eingeführt. Die wässrige Natronlauge entstammt einer Chloralkalielektrolyse. In der mehrstufigen Aufkonzentriervorrichtung (4) wird die wässrige Natronlauge (5) durch Verdampfen von Wasser aufkonzentriert und verlässt diese Vorrichtung (4) als wässrige Natronlauge (6) mit einer Konzentration von 50 Gew. % oder höher. Nicht dargestellt in dieser Prinzipskizze ist, dass ein Teil der für das Beheizen der ersten Stufe der mehrstufigen Aufkonzentriervorrichtung (4) benötigten Wärme aus Abwärme stammt, welche in weiteren Stufen der mehrstufigen Aufkonzentriervorrichtung (4) anfällt. Die in Figur 2 dargestellte Vorrichtung lässt sich vorzugsweise beim Bau neuer DCE-Anlagen realisieren, die mit Chloralkalianlagen gekoppelt sind.

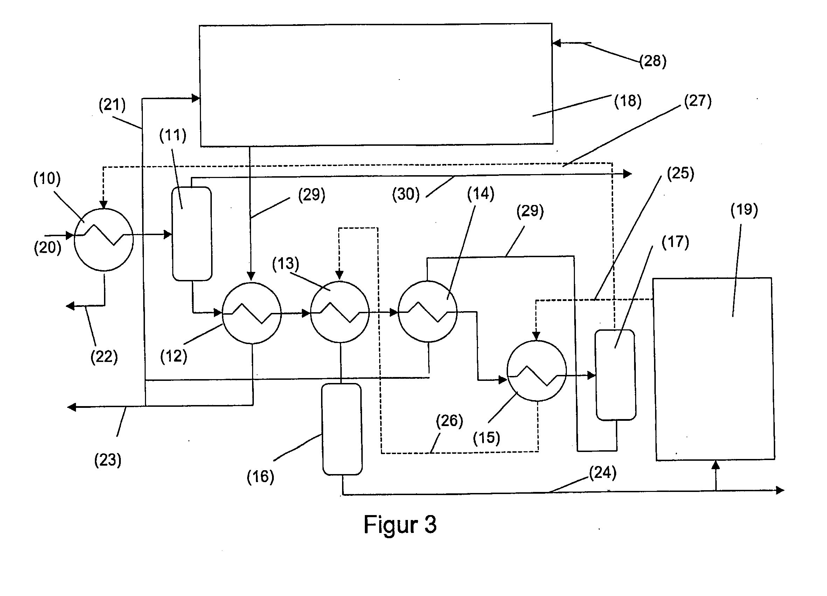

In Figur 3 wird eine mehrstufige Vorrichtung zum Aufkonzentrieren von wässriger Lauge beschrieben. Diese Vorrichtung kann vorzugsweise zur Nachrüstung von Anlagenverbunden bestehend aus einer DCE-Anlage und einer Chloralkalielektrolyseanlage eingesetzt werden, in denen bereits eine konventionelle Aufkonzentriervorrichtung für wässrige Lauge vorhanden ist, so wie in Figur 1 dargestellt. Die mehrstufige Vorrichtung umfasst eine bereits bestehende DCE-Anlage (19), eine bereits bestehende konventionelle Aufkonzentriervorrichtung (18) für wässrige Lauge und eine nachgerüstente mehrstufige Aufkonzentriervorrichtung für wässrige Lauge. Die konventionelle Aufkonzentriervorrichtung (18) wird hauptsächlich mit Hochdruck-Heissdampf (28) betrieben.

Die in Figur 3 dargestellte nachgerüstete Aufkonzentriervorrichtung besteht aus zwei Aufkonzentriereinheiten. Die erste Aufkonzentriereinheit wird von einem Plattenwärmetauscher (10) und einem Phasentrenner (11) gebildet, der durch Wasserdampf (27) aus der zweiten Aufkonzentrier-einheit beheizt wird. Die zweite Aufkonzentiereinheit besteht ebenfalls aus einem Plattenwärmetauscher (15) und einem Phasentrenner (17). Der Plattenwärmetauscher (15) wird durch DCE-Dampf (25) beheizt, welcher aus der DCE-Anlage (19) stammt. Zwischen der ersten und der zweiten Aufkonzentriereinheit ist eine Aufwärmeinheit für das Laugekonzentrat vorgesehen, die aus drei in Serie geschalteten Plattenwärmetauschern (12, 13, 14) besteht. Wärmetauscher (12) wird durch Heissdampf (29) beheizt, der aus der konventionellen Aufkonzentriervorrichtung (18) stammt. Nach dem Passieren des Wärmetauschers (12) wird das abgekühlte Kondensat (23) aus der Anlage ausgeschleust. Wärmetauscher (13) wird durch das Kondensat (26) des DCE-Dampfes (25) beheizt, das im Wärmetauscher (15) anfällt. Nach dem Passieren des Wärmetauschers (13) wird das abgekühlte Kondensat in den Sammelbehälter (16) geleitet und von dort aus als DCE-Strom (24) wieder in die DCE-Anlage (19) rückgeführt oder (teilweise) aus der Anlage ausgeschleust. Wärmetauscher (14) wird durch die aufkonzentrierte heiße Lauge (29) beheizt, die aus dem Phasentrenner (17) stammt. Nach dem Passieren des Wärmetauschers (14) wird die abgekühlte konzentrierte Lauge (21) in die konventionelle Aufkonzentriereinheit (18) geleitet, um dort weiter aufkonzentriert zu werden.

Die niedrig konzentrierte Natronlauge (20), z.B. 30 Gew.-%ig aus einer in der Figur 3 nicht dargestellten Chloralkalielektrolyseanlage wird in den Plattenwärmeaustauscher (10) gepumpt, dort erhitzt und in den Phasentrenner (11) übergeführt. Dort verdampft das Wasser teilweise und wird als Strom (30) aus der Anlage ausgeschleust, gegebenenfalls nach Wärmerückgewinnung durch einen nicht dargestellten Wärmetauscher. Die vorkonzentrierte Lauge verlässt den Phasentrenner (11) und wird in der Aufwärmeinheit bestehend aus drei Plattenwärmetauschern (12, 13, 14) weiter erhitzt, durchläuft sodann Wärmetauscher (15) und wird in den Phasentrenner (17) eingeleitet. Dort verdampft weiteres Wasser und der Wasserdampf (27) wird zum Beheizen des Wärmetauschers (10) verwendet und verlässt sodann als abgekühltes Kondensat (22) die Anlage. Die aufkonzentrierte Lauge wird aus Phasentrenner (17) abgepumpt und wird nach Durchlauf durch Wärmetauscher (14) zwecks weiterer Aufkonzentrierung in die Anlage (18) geleitet.

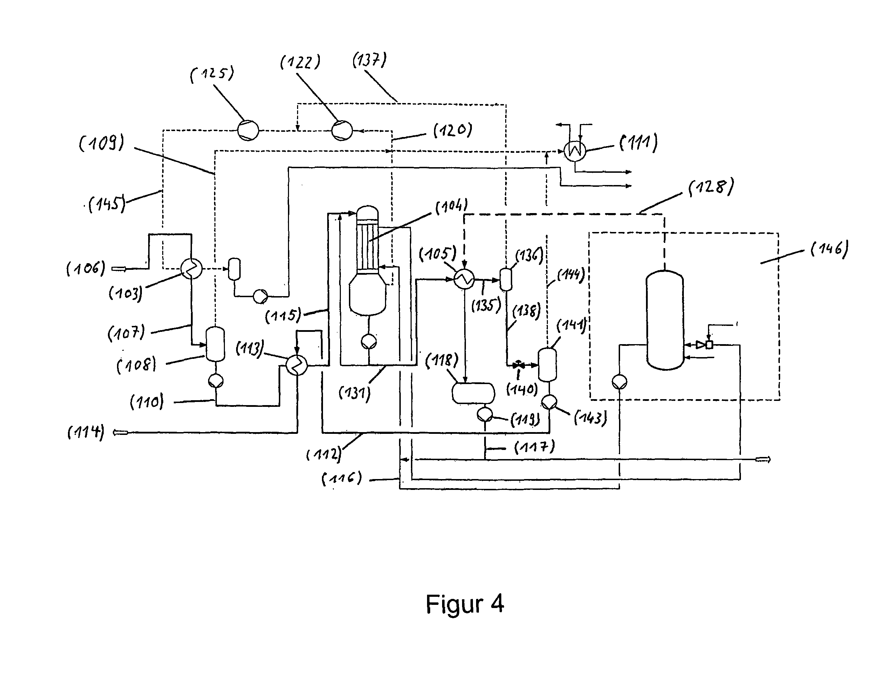

In Figur 4 wird eine weitere Vorrichtung zum Aufkonzentrieren von wässriger Lauge beschrieben. Diese Vorrichtung kann vorzugsweise in Neuanlagen eingesetzt werden oder in Anlagen in denen noch keine Verdampfereinheit für wässrige Lauge vorhanden ist.

Die in Figur 4 dargestellte Vorrichtung besteht aus drei Verdampfungsstufen. Die erste Verdampfungsstufe besteht aus einem Wärmetauscher (103) mit nachgeschaltetem Phasentrenner (108). Die zweite Verdampfungsstufe besteht aus einem Fallfilmverdampfer (104). Die dritte Verdampfungsstufe besteht aus einem weiteren Wärmetauscher (105) mit nachgeschaltetem Phasentrenner (136). Die zweite und die dritte Verdampfungsstufe werden durch den verwendbaren Teil der Reaktionshitze aus der EDC-Anlage, z.B. einer Direktchlorierungseinrichtung, beheizt. Die erste Verdampfungsstufe wird durch die Hitze, welche aus komprimiertem Wasserdampf, der bei der Verdampfung von Natronlauge entstanden ist, beheizt.

Natronlauge niedriger Konzentration, z.B. 30 Gew. %-ig und aus einer in Fig. 4 nicht dargestellten Chloralkalielektrolyseanlage stammend, tritt als Strom (106) in den Wärmetauscher (103) ein und wird dort aufgeheizt und unter Vakuum teilweise verdampft. Die aufgeheizte Natronlauge wird als Strom (107) in den Phasentrenner (108) übergeführt und dort in Wasserdampf (109) und vorkonzentrierte Natronlauge (110) getrennt. Der Wasserdampf (109) aus Phasentrenner (108) wird im Vakuumkondensator (111) kondensiert und das Kondensat wird aus der Anlage ausgeschleust.

Die vorkonzentrierte Natronlauge (110) wird nun weiter aufgeheizt, indem die aus dem Endprodukt konzentrierte Natronlauge (112) (z.B. 50 Gew. %-ige Natronlauge) stammende Wärme verwendet wird. Dazu werden die Ströme von Natronlauge (110, 112) in einen Wärmetauscher (113) geführt, wodurch eine Aufheizung der vorkonzentrierten Natronlauge (110) und eine Abkühlung der konzentrierten Natronlauge (112) erfolgt. Die abgekühlte konzentrierte Natronlauge wird als Strom (114) aus der Anlage ausgeschleust. Die aufgeheizte vorkonzentrierte Natronlauge wird als Strom (115) in einen Fallfillverdampfer (104) der zweiten Verdampfereinheit eingebracht. Fallfilmverdampfer (104) wird durch flüssiges DCE (Zirkulationsstrom (116)) beheizt, der aus der DCE-Anlage (146) stammt. Ein Teil von aus der dritten Verdampfungsstufe stammendem kondensiertem DCE (117) wird aus dem Sammelbehälter für kondensiertes DCE (118) durch DCE Pumpe (119) addiert. Durch diese Maßnahme kann die fühlbare Wärme aus dem kondensierten DCE aus der dritten Verdampfungsstufe ebenfalls genutzt werden. Der Wasserdampf (120) aus der zweiten Verdampfungsstufe (104) wird in einer Dampf-Kompressionseinheit (122) komprimiert.

Die dritte Verdampfungsstufe wird durch indirekten Wärmeaustausch betrieben. DCE-Dampf (128) aus der DCE-Anlage (146), beispielsweise aus einer Direktchlorierungsanlage, wird im Wärmetauscher (105) kondensiert. Durch die Kondensation des DCE-Dampfes (128) wird die vorkonzentrierte Natronlauge (131) aus der zweiten Verdampfungsstufe (104) im Wärmetauscher (105) weiter aufgeheizt und konzentriert. Die konzentrierte Natronlauge (135) aus Wärmetauscher (105) wird im Phasentrenner (136) vom Wasserdampf befreit. Wasserdampf (137) aus Phasentrenner (136) wird in einer Dampf-Kompressionseinheit (125) komprimiert. Aus Phasentrenner (136) fließt die konzentrierte Natronlauge (138) durch Schwerkraftwirkung über ein Regelventil (140) nach Phasentrenner (141). Die konzentrierte Natronlaugelösung (112) von ca. 50 Gew. % NaOH aus Phasentrenner (141) wird durch die Pumpe (143) über Wärmetauscher (113) aus der Anlage abgepumpt. Der in Phasentrenner (141) durch abgetrennte Wasserdampf (144) wird im Vakuumkondensator (111) kondensiert. Der komprimierte Dampf (145) dient zum Erhitzen des Wärmetauschers (103) der ersten Verdampfungsstufe.

## Patentansprüche

1. Verfahren zur Nutzung der Reaktionswärme aus der Herstellung von 1,2-Dichlorethan zum Aufkonzentrieren von wässriger Lauge, **dadurch gekennzeichnet, dass** die wässrige Lauge in mehreren Stufen aufkonzentriert wird, dass zumindest ein Teil der für das Aufkonzentrieren der wässrigen Lauge benötigten Wärme aus einer Anlage zur Herstellung von 1,2-Dichlorethan stammt und dass zumindestens ein weiterer Teil der für das Aufkonzentrieren der wässrigen Lauge benötigten Wärme aus mindestens einer der höheren Stufen der Anlage zum Aufkonzentrieren der wässrigen Lauge stammt und zum teilweisen Aufheizen der ersten Stufe verwendet wird, wobei das Verfahren mindestens zweistufig betrieben wird und folgende Schritte umfasst:
i) Erzeugung eines ersten wässrigen Laugenkonzentrats aus einer verdünnten wässrigen Lauge in einer ersten Aufkonzentriereinheit,
ii) Aufkonzentrieren des ersten wässrigen Laugenkonzentrats zu einem zweiten wässrigen Laugenkonzentrat in einer zweiten Aufkonzentriereinheit,
iii) Betrieb der zweiten Aufkonzentriereinheit zumindestens teilweise mit Wärme, die aus einer Anlage zur Herstellung von 1,2-Dichlorethan stammt, und
iv) Betrieb der ersten Aufkonzentriereinheit zumindestens teilweise mit Wärme, die aus der zweiten Aufkonzentriereinheit stammt, und wobei
zwischen erster und zweiter Aufkonzentriereinheit eine Aufwärmeinheit für das erste wässrige Laugenkonzentrat geschaltet ist, die durch Wärme beheizt wird, die aus der Anlage zur Herstellung von 1,2-Dichlorethan stammt, und/oder die durch Wärme beheizt wird, die aus der zweiten Aufkonzentriereinheit stammt, und/oder die durch Wärme beheizt wird, die aus einer Anlage zum Aufkonzentrieren von wässriger Lauge stammt, die mit einer Anlage zur Chloralkalielektrolyse kombiniert ist.

2. Verfahren nach Anspruch 1, **dadurch gekennzeichnet, dass** die eingesetzte wässrige Lauge aus einer Chloralkalielektrolyseanlage stammt.

3. Verfahren nach Anspruch 1, **dadurch gekennzeichnet, dass** die Aufwärmeinheit für das erste wässrige Laugenkonzentrat aus ein bis drei in Serie geschalteten Wärmetauschern besteht.

4. Verfahren nach Anspruch 1, **dadurch gekennzeichnet, dass** das Verfahren dreistufig betrieben wird und folgende Schritte umfasst:
i) Erzeugung eines ersten wässrigen Laugenkonzentrats aus einer verdünnten wässrigen Lauge in einer ersten Aufkonzentriereinheit,
ii) Aufkonzentrieren des ersten wässrigen Laugenkonzentrats zu einem zweiten wässrigen Laugenkonzentrat in einer zweiten Aufkonzentriereinheit,
iii) Aufkonzentrieren des zweiten wässrigen Laugenkonzentrats zu einem dritten wässrigen Laugenkonzentrat in einer dritten Aufkonzentriereinheit,
iv) Betrieb der zweiten und dritten Aufkonzentriereinheit zumindestens teilweise mit Wärme, die aus einer Anlage zur Herstellung von 1,2-Dichlorethan stammt, und
v) Betrieb der ersten Aufkonzentriereinheit zumindestens teilweise mit Wärme, die aus der zweiten und der dritten Aufkonzentriereinheit stammt.

5. Verfahren nach einem der Ansprüche 1 bis 4, **dadurch gekennzeichnet, dass** als erste Aufkonzentriereinheit ein oder mehrere in Reihe geschaltete Wärmetauscher oder Verdampfer verwendet werden, wobei mindestens 50 % der in der ersten Aufkonzentriereinheit benötigten thermischen Energie aus der oder den nachgeschalteten Aufkonzentriereinheit(en) stammt und durch den Einsatz von Wärmetauschern gewonnene thermische Energie ist und/oder thermische Energie ist, die aus wieder komprimiertem Wasserdampf, der beim Betrieb der nachgeschalteten Aufkonzentriereinrichtungen angefallen ist, gewonnen wurde.

6. Verfahren nach einem der Ansprüche 1 bis 4, **dadurch gekennzeichnet, dass** als zweite Aufkonzentriereinheit Fallfilmverdampfer verwendet werden, wobei mindestens 50 % der in der zweiten Aufkonzentriereinheit benötigten thermischen Energie aus dem DCE-Zirkulationsstrom gewonnen wird, der aus der DCE-Anlage stammt.

7. Verfahren nach Anspruch 4, **dadurch gekennzeichnet, dass** als dritte Aufkonzentriereinheit Wärmetauscher oder Verdampfer verwendet werden, wobei mindestens 50 % der in der dritten Aufkonzentriereinheit benötigten thermischen Energie aus DCE-Dampf gewonnen werden, der aus einer DCE-Anlage stammt.

8. Verfahren nach Anspruch 7, **dadurch gekennzeichnet, dass** ein Teil des aus der dritten Aufkonzentriereinheit stammenden kondensierten DCE zur Beheizung der zweiten Aufkonzentriereinheit abgezweigt wird und mit dem direkt aus der DCE-Anlage stammenden DCE-Zirkulationsstrom kombiniert wird.

9. Vorrichtung zum Aufkonzentrieren von wässriger Lauge, die mit einer Anlage zur Herstellung von 1,2-Dichlorethan gekoppelt ist und welche die folgenden Elemente umfasst:
A) erste Aufkonzentriereinheit zum Aufkonzentrieren von wässriger Lauge und zur Erzeugung eines ersten wässrigen Laugekonzentrats, die mit Abwärme aus einer zweiten Aufkonzentriereinheit beheizt wird,
B) zweite Aufkonzentriereinheit zum Aufkonzentrieren des ersten wässrigen Laugekonzentrats und zur Erzeugung eines zweiten wässrigen Laugekonzentrats, welche zumindestens teilweise mit Wärme beheizt wird, die aus einer Anlage zur Herstellung von 1,2-Dichlorethan stammt, und
C) zusätzlich neben den Elementen A) und B) eine Aufwärmeinheit C) für das erste wässrige Laugenkonzentrat, die zwischen die erste und die zweite Aufkonzentriereinheit geschaltet ist.

10. Vorrichtung nach Anspruch 9, **dadurch gekennzeichnet, dass** Aufwärmeinheit C) aus drei in Serie geschalteten Wärmetauschern besteht, wovon ein Wärmetauscher durch Wärme beheizt wird, die aus einer Anlage zur Herstellung von 1,2-Dichlorethan stammt, ein weiterer Wärmetauscher durch Wärme beheizt wird, die aus der zweiten Aufkonzentriereinheit stammt, und ein weiterer Wärmetauscher durch Wärme beheizt wird, die aus einer Anlage zum Aufkonzentrieren von wässriger Lauge stammt, die mit einer Anlage zur Chloralkalielektrolyse kombiniert ist.

11. Vorrichtung nach einem der Ansprüche 9 bis 10, **dadurch gekennzeichnet, dass** diese zur Nachrüstung mit einem bestehenden Verbund aus DCE-Anlage und Chloralkalielektrolyseanlage verschaltet wird.

12. Vorrichtung nach Anspruch 9, **dadurch gekennzeichnet, dass** diese zusätzlich zu den Elementen A), B) und C) die folgenden Elemente umfasst:
D) dritte Aufkonzentriereinheit zum Aufkonzentrieren des zweiten wässrigen Laugekonzentrats, welche zumindestens teilweise mit Wärme beheizt wird, die aus einer Anlage zur Herstellung von 1,2-Dichlorethan stammt, und gegebenenfalls
E) eine Wärmetauschereinheit zur Rückgewinnung von Wärme aus der dritten Aufkonzentriereinheit, welche mit der zweiten Aufkonzentriereinheit so verschaltet ist, dass die rückgewonnene Wärme zur Vorwärmung des ersten wässrigen Laugekonzentrats vor der zweiten Aufkonzentriereinheit genutzt werden kann.

13. Vorrichtung nach Anspruch 12, **dadurch gekennzeichnet, dass** die erste Aufkonzentriereinheit und die dritte Aufkonzentriereinheit jeweils ein oder mehrere in Serie geschaltete Wärmetauscher oder Verdampfer sind und worin die zweite Aufkonzentriereinheit ein Fallfilmverdampfer ist.

14. Vorrichtung nach Anspruch 12, **dadurch gekennzeichnet, dass** die erste und dritte Wärmetauschereinheit aus ein oder mehreren Plattenwärmetauschern gebildet wird.

15. Vorrichtung nach einem der Ansprüche 12 bis 14, **dadurch gekennzeichnet, dass** die erste Aufkonzentrierereinheit mit Wasserdampf aus einer Dampf-Kompressionseinheit beheizt wird, welche aus der zweiten Aufkonzentriereinheit und gegebenenfalls aus weiteren Aufkonzentriereinheiten stammenden Wasserdampf komprimiert.

## Claims

1. Method of utilizing the heat of reaction from the formation of 1,2-dichloroethane for concentrating aqueous alkali, **characterized in that** the aqueous alkali is concentrated in a plurality of stages, **in that** at least part of the heat required for concentrating the aqueous alkali originates from a plant for preparing 1,2-dichloroethane and **in that** at least a further part of the heat required for concentrating the aqueous alkali originates from at least one of the higher stages of the plant for concentrating the aqueous alkali and is used for the partial heating of the first stage, wherein the method is operated in at least two stages and comprises the following steps:
i) production of a first aqueous alkali concentrate from a dilute aqueous alkali in a first concentration unit,
ii) concentration of the first aqueous alkali concentrate to give a second aqueous alkali concentrate in a second concentration unit,
iii) operation of the second concentration unit at least partially by means of heat originating from a plant for preparing 1,2-dichloroethane and
iv) operation of the first concentration unit at least partially by means of heat originating from the second concentration unit, and wherein a heating unit for the first aqueous alkali concentrate is present between first and second concentration units, which is heated by heat originating from the plant for preparing 1,2-dichloroethane and/or is heated by heat originating from the second concentration unit and/or is heated by heat originating from a plant for concentrating aqueous alkali which is combined with a plant for chloralkali electrolysis.

2. Method according to Claim 1, the aqueous alkali used originates from a chloralkali electrolysis plant.

3. Method according to Claim 1, **characterized in that** the heating unit for the first aqueous alkali concentrate comprises from one to three heat exchangers connected in series.

4. Method according to Claim 1, **characterized in that** the method is operated in three stages and comprises the following steps:
i) production of a first aqueous alkali concentrate from a dilute aqueous alkali in a first concentration unit,
ii) concentration of the first aqueous alkali concentrate to give a second aqueous alkali concentrate in a second concentration unit,
iii) concentration of the second aqueous alkali concentrate to give a third aqueous alkali concentrate in a third concentration unit,
iv) operation of the second and third concentration units at least partially by means of heat originating from a plant for preparing 1,2-dichloroethane and
v) operation of the first concentration unit at least partially by means of heat originating from the second and third concentration units.

5. Method according to any of Claims 1 to 4, **characterized in that** one or more heat exchangers or evaporators connected in series is/are used as first concentration unit, where at least 50% of the thermal energy required in the first concentration unit originates from the downstream concentration unit(s) and is thermal energy recovered by the use of heat exchangers and/or thermal energy obtained from recompressed steam obtained in the operation of the downstream concentration apparatuses.

6. Method according to any of Claims 1 to 4, **characterized in that** falling film evaporators are used as second concentration unit, where at least 50% of the thermal energy required in the second concentration unit is obtained from the DCE circulation stream originating from the DCE plant.

7. Method according to Claim 4, **characterized in that** heat exchangers or evaporators are used as third concentration unit, where at least 50% of the thermal energy required in the third concentration unit is obtained from DCE vapor originating from a DCE plant.

8. Method according to Claim 7, **characterized in that** part of the condensed DCE originating from the third concentration unit is branched off for heating the second concentration unit and is combined with the DCE circulation stream coming directly from the DCE plant.

9. Apparatus for concentrating aqueous alkali, which is coupled with a plant for preparing 1,2-dichloroethane and comprises the following elements:
A) a first concentration unit for concentrating aqueous alkali and producing a first aqueous alkali concentrate, which is heated by means of waste heat from a second concentration unit,
B) a second concentration unit for concentrating the first aqueous alkali concentrate and producing a second aqueous alkali concentrate, which is heated at least partially by means of heat originating from a plant for preparing 1,2-dichloroethane, and
C) additionally, in addition to the elements A) and B), a heating unit C) for the first aqueous alkali concentrate which is located between the first and second concentration units.

10. Apparatus according to Claim 9, **characterized in that** heating unit C) comprises three heat exchangers connected in series, of which one heat exchanger is heated by heat originating from a plant for preparing 1,2-dichloroethane, a further heat exchanger is heated by heat originating from the second concentration unit and a further heat exchanger is heated by heat originating from a plant for concentrating aqueous alkali which is combined with a plant for chloralkali electrolysis.

11. Apparatus according to any of Claims 9 to 10, **characterized in that** it is retrofitted in an existing integrated plant made up of a DCE plant and a chloralkali electrolysis plant.

12. Apparatus according to Claim 9, wherein it comprises, in addition to the elements A), B) and C), the following elements:
D) a third concentration unit for concentrating the second aqueous alkali concentrate, which is operated at least partially by means of heat originating from a plant for preparing 1,2-dichloroethane, and optionally
E) a heat exchanger unit for recovering heat from the third concentration unit, which is connected to the second concentration unit in such a way that the recovered heat can be utilized for preheating the first aqueous alkali concentrate upstream of the second concentration unit.

13. Apparatus according to Claim 12, **characterized in that** the first concentration unit and the third concentration unit are each one or more heat exchangers or evaporators connected in series and the second concentration unit is a falling film evaporator.

14. Apparatus according to Claim 12, **characterized in that** the first and third heat exchanger units are each formed by one or more plate heat exchangers.

15. Apparatus according to any of Claims 12 to 14, **characterized in that** the first concentration unit is heated by means of steam from a steam compression unit which compresses steam originating from the second concentration unit and optionally from further concentration units.

## Revendications

1. Procédé pour utiliser la chaleur de réaction provenant de la préparation de 1,2-dichloréthane pour la concentration d'une lessive aqueuse, **caractérisé en ce que** la lessive aqueuse est concentrée en plusieurs étages, **en ce qu'**au moins une partie de la chaleur nécessaire pour la concentration de la lessive aqueuse provient d'une installation pour la préparation de 1,2-dichloroéthane et **en ce qu'**au moins une autre partie de la chaleur nécessaire pour la concentration de la lessive aqueuse provient d'au moins un des étages supérieurs de l'installation pour la concentration de la lessive aqueuse et est utilisée pour chauffer partiellement le premier étage, le procédé étant réalisé au moins à deux étages et comprenant les étapes suivantes :
i) production d'un premier concentrat de lessive aqueuse à partir d'une lessive aqueuse diluée dans une première unité de concentration,
ii) concentration du premier concentrat de lessive aqueuse en un deuxième concentrat de lessive aqueuse dans une deuxième unité de concentration,
iii) fonctionnement de la deuxième unité de concentration au moins partiellement avec de la chaleur qui provient d'une installation pour la préparation de 1,2-dichloroéthane et
iv) fonctionnement de la première unité de concentration au moins partiellement avec de la chaleur qui provient de la deuxième unité de concentration et
une unité de chauffage pour le premier concentrat de lessive aqueuse étant disposée entre la première et la deuxième unité de concentration, qui est chauffée par de la chaleur qui provient de l'installation pour la préparation de 1,2-dichloroéthane et/ou qui est chauffée par de la chaleur qui provient de la deuxième unité de concentration et/ou qui est chauffée par de la chaleur qui provient d'une installation pour la concentration de lessive aqueuse, qui est combinée à une installation pour l'électrolyse chlore-alcali.

2. Procédé selon la revendication 1, **caractérisé en ce que** la lessive aqueuse utilisée provient d'une installation d'électrolyse chlore-alcali.

3. Procédé selon la revendication 1, **caractérisé en ce que** l'unité de chauffage pour le premier concentrat de lessive aqueuse est constituée par un à trois échangeurs thermiques commutés en série.

4. Procédé selon la revendication 1, **caractérisé en ce que** le procédé est réalisé en trois étages et comprend les étapes suivantes :
i) production d'un premier concentrat de lessive aqueuse à partir d'une lessive aqueuse diluée dans une première unité de concentration,
ii) concentration du premier concentrat de lessive aqueuse en un deuxième concentrat de lessive aqueuse dans une deuxième unité de concentration,
iii) concentration du deuxième concentrat de lessive aqueuse en un troisième concentrat de lessive aqueuse dans une troisième unité de concentration,
iv) fonctionnement de la deuxième et de la troisième unité de concentration au moins partiellement avec de la chaleur qui provient d'une installation pour la préparation de 1,2-dichloroéthane et
v) fonctionnement de la première unité de concentration au moins partiellement avec de la chaleur qui provient de la deuxième et de la troisième unité de concentration.

5. Procédé selon l'une quelconque des revendications 1 à 4, **caractérisé en ce qu'**on utilise, comme première unité de concentration, un ou plusieurs échangeurs thermiques ou évaporateurs commutés en série, au moins 50% de l'énergie thermique nécessaire dans la première unité de concentration provenant de la ou des unité(s) de concentration disposée(s) en aval et étant de l'énergie thermique obtenue par l'utilisation d'échangeurs thermiques et/ou étant de l'énergie thermique qui a été obtenue à partir de vapeur d'eau à nouveau comprimée qui est obtenue lors du fonctionnement des dispositifs de concentration disposés en aval.

6. Procédé selon l'une quelconque des revendications 1 à 4, **caractérisé en ce qu'**on utilise, comme deuxième unité de concentration, des évaporateurs à film tombant, au moins 50% de l'énergie thermique nécessaire dans la deuxième unité de concentration provenant d'un flux en circulation de DCE qui provient de l'installation de DCE.

7. Procédé selon la revendication 4, **caractérisé en ce qu'**on utilise, comme troisième unité de concentration, des échangeurs thermiques ou des évaporateurs, au moins 50% de l'énergie thermique nécessaire dans la troisième unité de concentration étant obtenus à partir de vapeur de DCE qui provient d'une installation de DCE.

8. Procédé selon la revendication 7, **caractérisé en ce qu'**une partie du DCE condensé, provenant de la troisième unité de concentration, est déviée pour chauffer la deuxième unité de concentration et est combinée avec le flux en circulation de DCE provenant directement de l'installation de DCE.

9. Dispositif pour la concentration d'une lessive aqueuse, qui est couplé à une installation pour la préparation de 1,2-dichloroéthane et qui présente les éléments suivantes :
A) une première unité de concentration pour la concentration d'une lessive aqueuse et pour produire un premier concentrat de lessive aqueuse, qui est chauffée avec la chaleur perdue provenant d'une deuxième unité de concentration,
B) une deuxième unité de concentration pour la concentration du premier concentrat de lessive aqueuse et pour produire un deuxième concentrat de lessive aqueuse, qui est chauffée au moins partiellement avec de la chaleur qui provient d'une installation pour la préparation de 1,2-dichloroéthane et
C) en outre, à côté des éléments A) et B), une unité de chauffage C) pour le premier concentrat de lessive aqueuse, qui est commutée entre la première et la deuxième unité de concentration.

10. Dispositif selon la revendication 9, **caractérisé en ce que** l'unité de chauffage C) est constituée par trois échangeurs thermiques commutés en série, un échangeur thermique étant chauffé par de la chaleur qui provient d'une installation pour la préparation de 1,2-dichloroéthane, un autre échangeur thermique étant chauffé par de la chaleur qui provient de la deuxième unité de concentration et un autre échangeur thermique étant chauffé par de la chaleur qui provient d'une installation pour la concentration de lessive aqueuse, qui est combinée à une installation pour l'électrolyse chlore-alcali.

11. Dispositif selon l'une quelconque des revendications 9 à 10, **caractérisé en ce qu'**il est commuté pour la mise à niveau avec un ensemble existant d'une installation de DCE et d'une installation d'électrolyse chlore-alcali.

12. Dispositif selon la revendication 9, **caractérisé en ce qu'**il comprend, en plus des éléments A), B) et C), les éléments suivantes :
D) une troisième unité de concentration pour la concentration du deuxième concentrat de lessive aqueuse, qui est chauffée au moins partiellement avec de la chaleur qui provient d'une installation pour la préparation de 1,2-dichloroéthane et le cas échéant
E) une unité échangeuse de chaleur pour récupérer la chaleur de la troisième unité de concentration, qui est commutée avec la deuxième unité de concentration de manière telle que la chaleur récupérée peut être utilisée pour le préchauffage du premier concentrat de lessive aqueuse en amont de la deuxième unité de concentration.

13. Dispositif selon la revendication 12, **caractérisé en ce que** la première unité de concentration et la troisième unité de concentration sont à chaque fois un ou plusieurs échangeurs thermiques ou évaporateurs commutés en série et dans lequel la deuxième unité de concentration est un évaporateur à film tombant.

14. Dispositif selon la revendication 12, **caractérisé en ce que** la première et la troisième unité échangeuse de chaleur sont formées par un ou plusieurs échangeurs thermiques à plaques.

15. Dispositif selon l'une quelconque des revendications 12 à 14, **caractérisé en ce que** la première unité de concentration est chauffée avec de la vapeur d'eau provenant d'une unité de compression de vapeur, qui comprime la vapeur d'eau provenant de la deuxième unité de concentration et le cas échéant d'autres unités de concentration.
